# EUROPÄISCHE PATENTSCHRIFT

(11) **EP 2 413 798 B1**
(45) Veröffentlichungstag und Bekanntmachung des Hinweises auf die Patenterteilung: **23.01.2013**
(21) Anmeldenummer: 10710280.8
(22) Anmeldetag: 17.03.2010
(51) Int. Cl.: A61B 5/151

(54) **STECHHILFE MIT LANZETTENAUSWURFMITTEL**
PRICKING AID COMPRISING A LANCET EJECTION MEANS
MATÉRIEL DE PIQÛRE À DISPOSITIF D'ÉJECTION DE LANCETTE

(30) Priorität: 03.04.2009 DE 102009016126; 18.06.2009 DE 102009025444
(43) Veröffentlichungstag der Anmeldung: 08.02.2012
(73) Patentinhaber: Gerresheimer Regensburg GmbH, 93047 Regensburg (DE)
(72) Erfinder: KNIE, Andreas, 93051 Regensburg (DE); BUTZ, Marion, 93049 Regensburg (DE); SCHMITT, Bernhard, 92442 Wackersdorf (DE)
(74) Vertreter: Hannke, Christian
(86) Internationale Anmeldenummer: PCT/EP2010/053423
(87) Internationale Veröffentlichungsnummer: WO 2010/112334

(56) Entgegenhaltungen:
- EP-A1- 1 614 383
- EP-A2- 1 504 718
- WO-A1-2009/022144
- US-A- 5 984 940
- US-A1- 2006 247 670

## Beschreibung

Die Erfindung betrifft eine Stechhilfe mit einem Lanzettenauswurfmittel, insbesondere einer Fixier- und/oder Ausgabeeinrichtung gemäß dem Oberbegriff des Patentanspruches 1.

Bisher war es üblich, eine Lanzette oder ein Lanzettenelement, das innerhalb einer Lanzettenaufnahmeeinrichtung innerhalb einer Blutlanzettenvorrichtung zum Anstechen von Haut für eine Blutentnahme angeordnet ist, bei einem Wechsel des Lanzettenelementes dieses von Hand aus dem Gehäuse bzw. der Lanzettenhalteeinrichtung der Blutlanzettenvorrichtung herauszuziehen. Hierbei ist es problematisch, dass zum einen die Gefahr einer ungewollten Verletzung besteht und zum anderen blutbehaftete Lanzettenelemente, von einem zuvor erfolgten Einstich, angefasst werden müssen.

Die EP 1 614 383 A1 zeigt eine Lanzettenvorrichtung, gemäß der eine Lanzette reibschlüssig von einer zylinderförmigen und mit einem Schlitz versehenen Aufnahme gehalten wird. Gemäß dem Gegenstand dieser Druckschrift besteht ein Lanzettenhalter aus einem ersten Anteil und einem gegenüber dem ersten Anteil beweglichen zweiten Anteil. Der erste Anteil kann somit als Lanzettenaufnahmeeinrichtung angesehen werden und der zweite Anteil kann als Fixier- und/oder Ausgabeeinrichtung bezeichnet werden. Ein Zusammenwirken des ersten Anteiles mit dem zweiten Anteil erfolgt gemäß dem Gegenstand dieser Druckschrift beispielsweise beim Einsetzen einer Lanzette in die Komponente bzw. den zweiten Anteil oder kann durch den Einsatz des Ausschiebemechanismus bewirkt werden. Der Ausschiebemechanismus ermöglicht eine manuelle Kraftaufbringung, wodurch nachgelagert eine Verschiebung des ersten Anteiles gegenüber dem zweiten Anteil des Lanzettenhalters erfolgt.

Die US 5 984 940 A zeigt ebenfalls eine Lanzette in einer im Wesentlichen zylinderförmigen Ausnehmung. Das Lanzettenelement ist gemäß dem Gegenstand dieser Druckschrift nach dem Abschrauben eines Kappenelementes über eine Vielzahl von Komponenten, insbesondere eine Ausschiebeplatte, einen Ausschiebestab sowie ein Auflageelement, ausschiebbar. einem Lanzettenelement. Die Kopplung ist bevorzugt rein reibschlüssig, es ist jedoch auch eine formschlüssige oder kombinierte Verbindung aus Formfluss und Reibfluss bzw. Kraftschluss vorstellbar.

Das mindestens eine Federelement dient zum Federkraftbeaufschlagen der Lanzettenaufnahmeeinrichtung, damit Energie für die Bewegung der Lanzetteaufnahmeeinrichtung bereit gestellt wird. Die Lanzettenaufnahmeeinrichtung ist mit einer Steuerkurve derart gekoppelt, dass durch eine Bewegung der Steuerkurve die Lanzettenaufnahmeeinrichtung bewegbar ist wobei die Steuerkurve mit dem mindestens einem Federelement gekoppelt ist. Durch Auslösen der Bewegung mittels des Betätigungselementes ist die Lanzettenaufnahmeeinrichtung derart bewegbar, dass sich das Lanzettenelement selbsttätig aus der Vorrichtung in einer Ausgaberichtung heraus bewegt oder bevorzugt aus der Vorrichtung heraus und wieder in einer Aufnahmerichtung in die Vorrichtung hineinbewegt.

Die Ausgaberichtung und die Aufnahmerichtung sind beide in Richtung einer Längsachse der Lanzettenaufnahmeeinrichtung orientiert, jedoch führt die Ausgaberichtung aus der Blutlanzettenvorrichtung heraus und die Aufnahmerichtung entgegen der Ausgaberichtung in die Blutlanzettenvorrichtung hinein.

Ferner ist ein Betätigungselement zum Auslösen einer Bewegung der Lanzettenaufnahmeeinrichtung vorgesehen, wobei das Betätigungselement bevorzugt als Druckknopf ausgeführt ist, der manuell betätigbar ist. Dieser Druckknopf wirkt bei einer Betätigung mit der Steuerkurve zusammen, wodurch die Steuerkurve aus einer arretierten Position gelöst wird, um eine Schwenkbewegung auszuführen. Die Lanzettenaufnahmeeinrichtung gleitet dabei auf der Steuerkurve ab und wird selbst in Bewegung versetzt.

Mittels der Fixier- und/oder Ausgabeeinrichtung ist die Kopplung zwischen der Lanzettenaufnahmeeinrichtung und dem Lanzettenelement veränderbar, d.h., dass bevorzugt zwischen einer reibschlüssigen Verbindung und einem entkoppelten Zustand (keiner Verbindung bzw. einer losen Verbindung) gewechselt werden kann.

Das Zusammenwirken der Fixier- und/oder Ausgabeeinrichtung mit der Lanzettenaufnahmeeinrichtung erfolgt beispielsweise mittels eines Kraftschlusses zwischen diesen Einrichtungen. Die Kraft ist hierbei beispielsweise direkt von der Fixier- und/oder Ausgabeeinrichtung auf die Lanzettenaufnahmeeinrichtung übertragbar oder vice versa.

Erfindungsgemäß ist mittels einer Bewegung der Fixier- und/oder Ausgabeeinrichtung in Längsrichtung der Lanzettenaufnahmeeinrichtung und mittels eines Eingreifens der Fixier- und/oder Ausgabeeinrichtung in mindestens einer Ausnehmung der Lanzettenaufnahmeeinrichtung die Lanzettenaufnahmeeinrichtung verformbar, wobei die Verformung bevorzugt rein elastisch erfolgt und eine Rückverformung bevorzugt ebenfalls selbsttätig erfolgt. D.h. bevorzugt liegt in einer ersten Position einer Fixier- und/oder Ausgabeeinrichtung ein unverformter Zustand der Lanzettenaufnahmeeinrichtung vor, der einen ersten Kopplungszustand darstellt, in dem das Lanzettenelement mit der Lanzettenaufnahmeeinrichtung ortsfest verbunden ist, und in der zweiten Position liegt bevorzugt ein verformter Zustand vor, der einen zweiten Kopplungszustand darstellt, in dem das Lanzettenelement relativ zu der Lanzettenaufnahmeeinrichtung bewegbar ist.

Es ist ebenfalls vorstellbar, dass das Lanzettenelement in dem zweiten Kopplungszustand nicht in Längsrichtung, sondern in einer Richtung die im Wesentlichen rechtwinklig zur Längsrichtung orientiert ist, bewegbar ist. Weiterhin ist eine kombinierte Bewegung des Lanzettenelements in Längsrichtung und rechwinklig zur Längsrichtung vorstellbar.

Der Gegenstand des Anspruches 1 ist vorteilhaft gegenüber den aus dem Stand der Technik bekannten Gegenständen, da der Auswurf eines Lanzettenelements erfolgen kann, ohne dass der Nutzer bzw. der Patient das gebrauchte Lanzettenelement anfassen muss.

In einer weiteren bevorzugten Ausführungsform der vorliegenden Erfindung ist ein Abschnitt der Fixier- und/oder Ausgabeeinrichtung keilförmig gestaltet. Dies bedeutet, dass sich der Querschnitt des keilförmigen Abschnittes zumindest in einer Richtung verändert. Durch ein Zusammenwirken des keilförmigen Abschnittes mit einem Abschnitt der Lanzettenaufnahmeeinrichtung ist eine Veränderung eines Querschnittes der Lanzettenaufnahmeeinrichtung durchführbar.

Die Form des Abschnittes der Lanzettenaufnahmeeinrichtung ist beliebig wählbar oder an die Form des keilförmigen Abschnittes entsprechend anpassbar. Diese Ausführungsform ist vorteilhaft, da durch den keilförmigen Abschnitt die Fixier- und/oder Ausgabeeinrichtung in präziser Weise mit dem Abschnitt der Lanzettenaufnahmeeinrichtung in Kontakt bringbar ist und die zur Verformung der Lanzettenaufnahmeeinrichtung benötigte Kraft in der gewünschten Richtung übertragbar ist.

In einer weiteren bevorzugten Ausführungsform der vorliegenden Erfindung ist die Lanzettenaufnahmeeinrichtung zumindest Abschnittsweise rohrförmig ausgebildet. Dies bedeutet, dass ein hohler Körper einer beliebigen und/oder variablen Querschnittsform wie z.B. eckig 3-, 4-, Mehreckig) oder kurvenförmig bevorzugt rund vorgesehen ist.

Der Abschnitt der Lanzettenaufnahmeeinrichtung ist im Bereich der mindestens ein Ausnehmung bevorzugt in der mindestens eine Ausnehmung ausgebildet, wodurch der keilförmige Abschnitt der Lanzettenhalteeinrichtung und/oder in der Lanzettenhalteeinrichtung bewegbar ist. Weiterhin bevorzugt sind mindestens zwei Ausnehmungen in der Lanzettenaufnahmeeinrichtung vorgesehen. Dies hat den Vorteil, dass die Lanzettenhalteeinrichtung einfacher verformbar ist. Im Falle zweier Ausnehmungen sind diese bevorzugt im Wesentlichen gegenüber voneinander angeordnet und zumindest eine Ausnehmung erstreckt sich zumindest abschnittsweise in Längsrichtung und ist schlitzartig ausgebildet. Dies hat ebenfalls den Vorteil, dass die Aufhebung der Verbindung z.B. des Kraftschlusses beispielsweise durch die Reduzierung der Reibkraft zwischen dem Lanzettenelement und der Lanzettenaufnahmeeinrichtung erfolgt.

In einer weiteren bevorzugten Ausführungsform der vorliegenden Erfindung ist der keilförmige Abschnitt der Fixier- und/oder Ausgabeeinrichtung in einer Ausbuchtung der Ausnehmung der Lanzettenaufnahmeeinrichtung einbringbar. Dies bedeutet, dass die Lanzettenaufnahmeeinrichtung eine Ausbuchtung aufweist, die zulässt, dass der keilförmige Abschnitt zumindest teilweise oder bevorzugt vollständig in die Ausnehmung einführbar ist. Der Schlitz stellt hierbei eine weitere Erstreckung der Ausbuchtung dar. Dies bedeutet, dass der Schlitz und die Ausbuchtung miteinander verbunden sind. Diese Ausführungsform ist vorteilhaft, da der keilförmige Abschnitt durch die Ausnehmung definiert in den Schlitz einführbar ist, woraus eine hohe Funktionssicherheit resultiert.

In einer weiteren bevorzugten Ausführungsform der vorliegenden Erfindung ist der keilförmige Abschnitt der Fixier- und/oder Ausgabeeinrichtung zumindest zeitweise d.h. im Kopplungszustand und abschnittsweise d.h. zumindest ein Bereich des keilförmigen Abschnittes mit dem Abschnitt der Lanzetteaufnahmeeinrichtung in Kontakt bringbar, wobei sich die Keilkante bevorzugt im Wesentlichen rechtwinklig zur Längsrichtung des Aufnahmeelements erstreckt. Ferner ist eine Kraft durch die Fixier- und/oder Ausgabeeinrichtung manuell auf das Lanzettenelement übertragbar. Dies bedeutet, dass das Lanzettenelement durch eine vom Nutzer bzw. Patienten aufgebrachte Kraft bewegbar ist, wobei durch das Aufbringen der Kraft zeitgleich das Lanzettenelement zumindest teilweise in der Ausgaberichtung bewegbar und der Schlitz veränderbar ist. Eine Veränderung des Schlitzes entspricht einer Aufweitung und/oder einer Reduzierung des Querschnitts der Lanzettenaufnahmeeinrichtung.

In einer weiteren bevorzugten Ausführungsform der vorliegenden Erfindung weist der keilförmige Abschnitt einen spitzenartigen Frontbereich auf, wobei spitzenartig hierbei einen Frontbereich beschreibt, der gegenüber einem Querschnitt eines weiteren Bereichs des keilförmigen Abschnitts eine geringere Fläche bzw. Querschnittsfläche aufweist. Der spitzenartige Frontbereich kann somit stumpf oder scharfkantig ausgebildet sein. Der Frontbereich ist ferner zumindest abschnittsweise in den Schlitz einführbar, wobei bevorzugt die Höhe des Frontbereichs geringer ist als die Schlitzhöhe. Weiterhin ist der Frontbereich des keilförmigen Abschnitts zumindest abschnittsweise in einen das Lanzettenelement aufnehmenden Innenraum der Lanzettenaufnahmeeinrichtung einführbar, wodurch die Fixier- und/oder Ausgabeeinrichtung mit dem Lanzettenelement in Kontakt bringbar ist. Diese Ausführungsform ist vorteilhaft, da sie ebenfalls durch den Kontakt zwischen dem sich zumindest abschnittsweise in den Innenraum der Lanzettenaufnahmeeinrichtung erstreckenden keilförmigen Abschnitt und dem Lanzettenelement eine Kraftübertragung von der Bedienperson über die Fixier- und/oder Ausgabeeinrichtung auf das Lanzettenelement ermöglicht, die wiederum ein zeitgleiches Verschieben des Lanzettenelements und eine Verformung der Lanzettenaufnahmeeinrichtung ermöglicht.

In einer weiteren bevorzugten Ausführungsform der vorliegenden Erfindung weist die Fixier- und/oder Ausgabeeinrichtung ein stabförmiges Element auf, an dessen Ende der keilförmige Abschnitt, bevorzugt seitlich versetzt, angeordnet ist. Dies bedeutet, dass bevorzugt ein Zentrum des keilförmigen Abschnittes versetzt zu einem Zentrum des stabförmigen Elements angeordnet ist. Diese Ausführungsform ist vorteilhaft, da eine Funktionstrennung bzw. Funktionsaufspaltung des keilförmigen Abschnittes derart erfolgt, dass der direkt am stabförmigen Element angeordnete Abschnitt des keilförmigen Abschnittes zur Aufnahme größerer Kräfte und somit zur Verformung der Lanzettenaufnahmeeinrichtung verwendbar ist, während der vom stabförmigen Element abstehende Abschnitt des keilförmigen Abschnittes lediglich zum Ausschieben bzw. Bewegen des Lanzettenelementes vorgesehen ist. Dies führt zu einer belastungsgerechten und optimierten Auslegung der Fixier- und/oder Ausgabeeinrichtung durchführbar ist, wodurch die Funktionssicherheit erhöht wird und Materialeinsparungen erfolgen können.

In einer weiteren bevorzugten Ausführungsform der vorliegenden Erfindung weist das keilförmige Element eine Unterseite auf, die mit einer Unterseite des stabförmigen Elements in einer gemeinsamen Ebene angeordnet ist. Diese Ausführungsform ist vorteilhaft, da der keilförmige Abschnitt das stabförmige Element mittels einer Wandung bzw. Stütze so abgestützt werden können, dass hohe Kräfte und Momente auf das stabförmige Element bzw. den keilförmigen Abschnitt wirken können, ohne das die Belastung zu einer Beschädigung der Fixier- und/oder Ausgabeeinrichtung führen würde.

In einer weiteren bevorzugten Ausführungsform der vorliegenden Erfindung weist der keilförmige Abschnitt neben dem spitzenartigen Frontbereich einen Endbereich auf, wobei die Querschnittshöhe des spitzenartigen Frontbereichs bevorzugt geringer ist als die Querschnittshöhe des stabförmigen Elements. Ferner ist die Querschnittshöhe des Endbereichs bevorzugt höher als die Querschnittshöhe des stabförmigen Elements, wobei auch denkbar ist, dass die Querschnittshöhe des Endbereichs der Querschnittshöhe des stabförmigen Elements entspricht oder geringer als die Querschnittshöhe des stabförmigen Elements ausgeführt ist. Die bevorzugte Ausführungsform ist vorteilhaft, da sie zu einer Reduzierung des Materialanteils und somit der Kosten und des Gewichts führt.

In einer weiteren bevorzugten Ausführungsform der vorliegenden Erfindung ist die Bewegung der Fixier- und/oder Ausgabeeinrichtung durch mindestens ein weiteres Federelement mit dem Gehäuse gekoppelt. Hieraus ergibt sich, dass die Kopplung des Federelements mit der Fixier- und/oder Ausgabeeinrichtung in Abhängigkeit von der Auslenkung des Federelements veränderbar ist. Das Federelement ist durch an der Fixier- und/oder Ausgabeeinrichtung und/oder dem Grundkörper vorgesehenen Mittel geführt und die Fixier- und/oder Ausgabeeinrichtung ist mittels dem Federelement entgegen der Ausgaberichtung bewegbar. Dies bedeutet, dass die Fixier- und/oder Ausgabeeinrichtung bei einer Verschiebung eine Veränderung der Kopplung mit dem Gehäuse bzw. dem Grundkörper erfährt.

Bevorzugt findet nach einer Freigabe der Fixier- und/oder Ausgabeeinrichtung durch den Nutzer bzw. Patienten eine Rückstellung der Fixier- und/oder Ausgabeeinrichtung statt, wodurch die Lanzettenaufnahmeeinrichtung von einem verformten Zustand in einen unverformten Zustand überführt wird. Diese Ausführungsform ist vorteilhaft, da der Schieber bevorzugt selbsttätig mittels des Federelementes in eine von dem Lanzettenaufnahmeelement beabstandete Position überführt wird und somit immer, wenn der Nutzer bzw. der Patient die Lanzettenvorrichtung freigibt, es stets ein unverformter Zustand der Lanzettenaufnahmeeinrichtung ermöglicht wird bzw. vorliegt.

In einer weiteren bevorzugten Ausführungsform der vorliegenden Erfindung ist an der Fixier- und/oder Ausgabeeinrichtung ein Anschlag vorgesehen, mittels dem der Verschiebeweg der Fixier- und/oder Ausgabeeinrichtung begrenzt ist und der mit bevorzugt einem entsprechend ausgebildeten bzw. korrespondieren Bereich des Grundkörpers zusammenwirkt. Dies hat den Vorteil, dass eine maximale Bewegung der Fixier- und/oder Ausgabeeinrichtung definiert vorgebbar ist. Dies hat wiederum den Vorteil, dass übermäßige Belastungen der Blutlanzettenvorrichtungsbauteile ausgeschlossen bzw. begrenzt sind.

In einer weiteren bevorzugten Ausführungsform der vorliegenden Erfindung ist auf einer Seite des Anschlages der Fixier- und/oder Ausgabeeinrichtung ein Handbestätigungselement vorgesehen. Mittels dieses Handbestätigungselementes lässt sich die Fixier- und/oder Ausgabeeinrichtung durch den Nutzer bzw. Patienten bewegen.

In einer weiteren bevorzugten Ausführungsform der vorliegenden Erfindung weist die Fixier- und/oder Ausgabeeinrichtung ein sich im Wesentlichen rechtwinklig zur Längsrichtung erstreckendes Trägerelement auf, an dem im Wesentlichen rechtwinklig das Mittel zum Führen des weiteren Federelementes, das stangen- bzw. stabartige Element und der Anschlag vorgesehen sind.

Bevorzugt sind das Mittel zum Führen des weiteren Federelements, das stangenartige Element und der Anschlag parallel zueinander angeordnet.

Ferner sind bevorzugt zwischen dem Anschlag und dem Mittel zum Führen des weiteren Federelementes und/oder zwischen dem stabförmigen Element und dem Mittel zum Führen des weiteren Federelementes Versteifungselemente vorgesehen. Diese Ausführungsform ist vorteilhaft, da auf Grund der Versteifungselemente vergleichsweise hohe Kräfte auf die zuvor genannte Elemente übertragen werden können, ohne das diese äußerst massiv ausgeführt werden müssen, d.h. Materialeinsparungen mittels optimierter Krafteinleitungen gegeben sind.

In einer weiteren bevorzugten Ausführungsform der vorliegenden Erfindung ist zumindest der keilförmige Abschnitt einstückig mit der übrigen Fixier- und/oder Ausgabeeinrichtung insbesondere mit dem stabförmigen Element hergestellt. Dies ist vorteilhaft, da aufgrund dieser Ausführung der Kraftfluss innerhalb der Fixier- und/oder Ausgabeeinrichtung optimal verlaufen kann, da keine Spannung aufgrund fügetechnischer Ungenauigkeiten auftreten.

In einer weiteren bevorzugten Ausführungsform der vorliegenden Erfindung weist der Grundkörper zumindest eine ebene Innenwandung zum definierten Verschieben der Fixier- und/oder Ausgabeeinrichtung auf. Diese Ausführungsform ist vorteilhaft, da die Fixier- und/oder Ausgabeeinrichtung auf der Innenwandung bzw. einem Steg oder einem Abschnitt einer Innenwandung verschoben werden kann und die Innenwandung, der Abschnitt der Innenwandung oder der Steg ebenfalls zur Aufnahme übermäßiger Betätigungskräfte dient, wodurch eine Beschädigung der Blutlanzettenvorrichtung verhindert wird.

An dieser Stelle sei noch darauf hingewiesen, dass sämtliche in den Anmeldungsunterlagen offenbarten Merkmale einzeln oder in Kombination miteinander gattungsgemäße oder aus dem Stand der Technik bekannte Blutlanzettenvorrichtungen vorteilhaft weiterentwickeln.

Weitere Vorteile, Ziele und Eigenschaften vorliegender Erfindung werden anhand nachfolgender Beschreibung anliegender Zeichnungen erläutert, in welcher beispielhaft Blutlanzettenvorrichtungen für die Untersuchung von Blut dargestellt sind. Bauteile der Blutlanzettenvorrichtung, welche in den Figuren wenigsten im Wesentlichen hinsichtlich ihrer Funktion übereinstimmen, können hierbei mit gleichen Bezugszeichen gekennzeichnet sein, wobei diese Bauteile nicht in allen Figuren beziffert oder erläutert sein müssen.

Darin zeigen:
- Fig. 1: Eine Darstellung einer erfindungsgemäßen Blutlanzettenvorrichtung;
- Fig. 2: eine offene Darstellung der erfindungsgemäßen Blutlanzettenvorrichtung in einem entkoppelten Zustand;
- Fig. 3: eine offene dreidimensionale Darstellung der erfindungsgemäßen Blutlanzetten vorrichtung in einem entkoppelten Zustand;
- Fig. 4: eine offene Darstellung einer erfindungsgemäßen Blutlanzettenvorrichtung in einem gekoppelten Zustand;
- Fig. 5: eine offene dreidimensionale Darstellung einer erfindungsgemäßen Blutlanzettenvorrichtung in einem gekoppelten Zustand;
- Fig. 6: eine offene Darstellung einer erfindungsgemäßen Blutlanzettenvorrichtung in einer Ausgabekonfiguration;
- Fig. 7: eine offene dreidimensionale Darstellung einer erfindungsgemäßen Blutlanzettenvorrichtung in einer Ausgabekonfiguration;
- Fig. 8: eine weitere perspektivische Darstellung einer erfindungsgemäßen Blutlanzettenvorrichtung in einem gekoppelten Zustand;
- Fig. 9: eine weitere perspektivische Darstellung einer erfindungsgemäßen Blutlanzettenvorrichtung in einer Ausgabekonfiguration;
- Fig.10: eine weitere Darstellung der Blutlanzettenvorrichtung mit arretierter Fixier- und/oder Ausgabeeinrichtung;
- Fig. 11: eine dreidimensionale Darstellung der Fixier- und/oder Ausgabeeinrichtung.

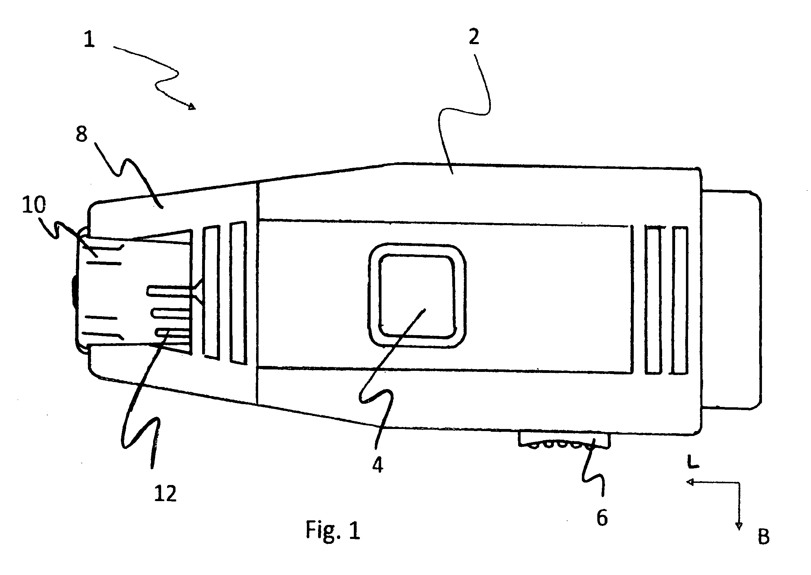

Fig. 1 zeigt eine Blutlanzettenvorrichtung 1 mit einem Grundkörper 2, der ein Betätigungselement 4 zum Auslösen der Funktion der Blutlanzettenvorrichtung 1 aufweist. Ein Handbetätigungselement 6 zum Auswurf von Lanzettenelementen (in dieser Darstellung nicht gezeigt) und einer Kappe 8, an der ein Stellrad 10 vorgesehen ist, mittels dem eine Einstellung der Einstechtiefe durchführbar ist. Die Einstechtiefe ist hierbei mittels Markierungen 12 an dem Stellrad 10 und der Kappe 8 in Stufen einstellbar. Die Blutlanzettenvorrichtung 1 erstreckt sich hierbei in Längsrichtung L und Breitenrichtung B.

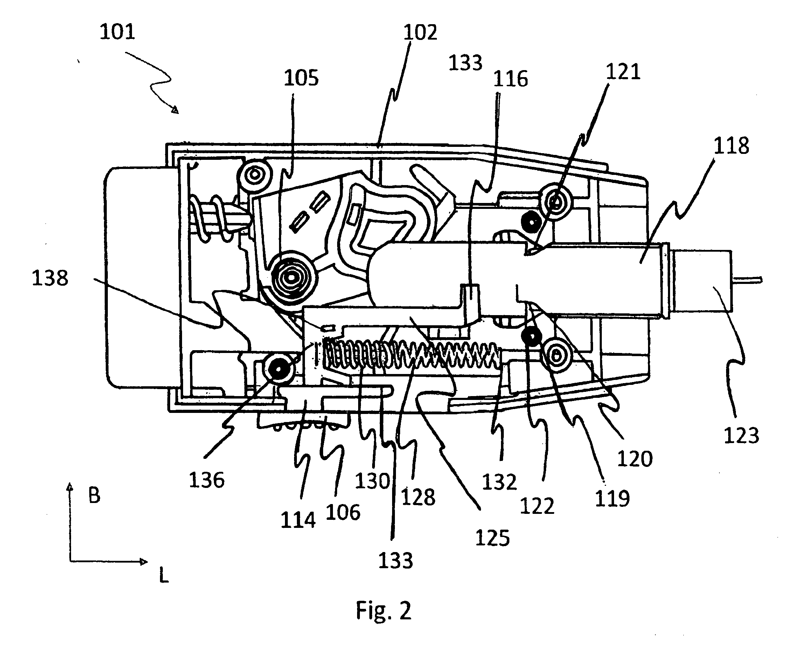

In Fig. 2 ist eine Blutlanzetteneinrichtung 101 gezeigt, die sich ebenfalls in Längsrichtung L und Breitenrichtung B erstreckt. Die Blutlanzettenvorrichtung 101 weist einen Grundkörper 102 auf, in dem eine Vielzahl an Vorrichtungskomponenten angeordnet sind. Mittels eines Federelementes 105 und einer Steuerkurve ist eine Kraft für die Funktionsausführung auf die Lanzettenaufnahmeeinrichtung 118 übertragbar.

Mittels eines Handbetätigungselementes 106, das an einer Fixer- und/oder Ausgabeeinrichtung 114 angeordnet ist bzw. mit dieser einstückig verbunden ist, lässt sich ein keilförmiger Abschnitt 116 in Längsrichtung L der Blutlanzettenvorrichtung 101 verschieben.

An der Lanzettenaufnahmeeinrichtung 118 ist ein Abschnitt 119 vorgesehen, an dem bzw. mit dem der keilförmige Abschnitt 116 in Eingriff bzw. in Kontakt gebracht werden kann. Insbesondere ist der Abschnitt 119 der Lanzettenaufnahmeeinrichtung 118 in einer Ausnehmung 120 der Lanzettenaufnahmeeinrichtung 118 vorgesehen.

Neben einer ersten Ausnehmung 120 weist die Lanzettenaufnahmeeinrichtung 118 ebenfalls eine zweite Ausnehmung 121 auf. In mindestens einer der beiden Ausnehmungen 120, 121 ist bevorzugt eine Ausbuchtung 122 vorgesehen. In diese Ausbuchtung 122 ist der keilförmige Abschnitt 116 zumindest abschnittsweise einbringbar. Ferner ist der keilförmige Abschnitt 116 der Ausbuchtung 122 definiert zuführbar.

In der Lanzettenaufnahmeeinrichtung 118 ist ein Lanzettenelement 123 anordenbar, weiterhin weist die Fixier- und/oder Ausgabeeinrichtung 114 ein stabförmiges Element 125 auf und ist bevorzugt zumindest abschnittsweise von einem weiteren Federelement 128 umgeben, das sich an einem Mittel der Fixier- und/oder Ausgabeeinrichtung abstützt bzw. dadurch geführt ist.

Dieses Mittel 130 der Fixier- und/oder Ausgabeeinrichtung 114 ist bevorzugt einstückig mit der restlichen Fixier- und/oder Ausgabeeinrichtung 114 ausgeführt und erstreckt sich im Wesentlichen zylinderförmig in Längsrichtung der Blutlanzettenvorrichtung 101. Das weitere Federelement 128 steht ferner mit an dem Grundkörper 102 angebrachten Mitteln 132 in Kontakt bzw. ist daran abgestützt.

Weiterhin umfasst die Fixier- und/oder Ausgabeeinrichtung 114 einen Anschlag 133, der ebenfalls bevorzugt einstückig mit der restlichen Fixier- und/oder Ausgabeeinrichtung 114 ausgeführt ist. Dieser Anschlag 133 dient dazu eine Begrenzung der Bewegung der Fixier- und/oder Ausgabeeinrichtung 114 in Längsrichtung L zu gewährleisten, es ist hierbei vorstellbar, dass eine Begrenzung der Verschiebebewegung der Fixier- und/oder Ausgabeeinrichtung 114 ebenfalls oder alternativ über eine weitere Einrichtung (nicht gezeigt) erfolgt.

Das Mittel 130 der Fixier- und/oder Ausgabeeinrichtung 114 sowie das stabförmige Element 125 sind im Wesentlichen rechtwinklig an einem Trägerelement 136 angeordnet. Dieses Trägerelement 136 ist ebenfalls ein Bestandteil der Fixier- und/oder Ausgabeeinrichtung 114. Weiterhin sind das Mittel 130 der Fixier- und/oder Ausgabeeinrichtung 114 sowie das stabförmige Element 125 mittels Versteifungselementen 138 mit dem Trägerelement 136 verbunden. Durch die Versteifungselemente 138 erfolgt zumindest abschnittsweise eine Erhöhung der Belastbarkeit der Fixier- und/oder Ausgabeeinrichtung 114.

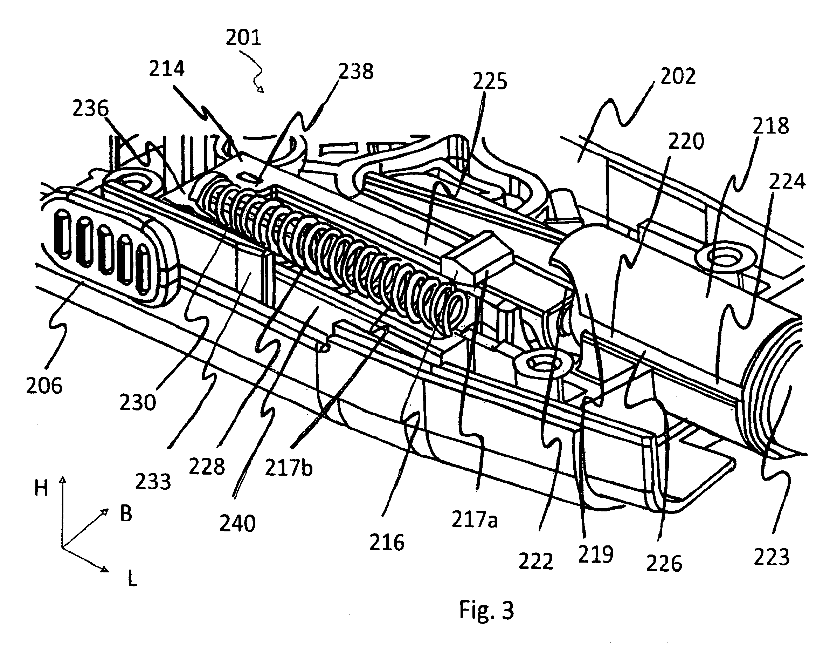

Fig. 3 zeigt in perspektivischer Darstellung einen Querschnitt einer Blutlanzettenvorrichtung 201, die sich in Längsrichtung L, Breitenrichtung B und Höhenrichtung H erstreckt. Die Blutlanzettenvorrichtung 201 weist ebenfalls einen Grundkörper 202 auf, in dem eine Vielzahl an Vorrichtungskomponenten angeordnet ist. Weiterhin ist ein Handbestätigungselement 206 vorgesehen, an dem reibungserhöhende und/oder formschlussgenerierende Erhebungen angeordnet sind.

Die Fixier- und/oder Ausgabeeinrichtung 214 weist einen keilförmigen Abschnitt 216 auf, an dem ein Frontbereich 217a und ein Endbereich 217b ausgestaltet ist. Der Frontbereich 217a hat eine geringere Querschnittsfläche als der Endbereich 217b.

Die Lanzettenaufnahmeeinrichtung 218 ist in einem ersten Abschnitt rohrförmig ausgebildet und in einem weiteren Abschnitt plattenförmig, wobei zwei Plattenelemente bevorzugt rechtwinklig zueinander angeordnet sind, um eine Erhöhung der Bauteilstabilität zu erhalten. Ferner steht die Lanzettenaufnahmeeinrichtung 218 über ein Verbindungselement mit einer Steuerkurve in Kontakt.

An einem Abschnitt 219 der Lanzettenaufnahmeeinrichtung 218 ist eine Ausnehmung 220 vorgesehen, die bevorzugt eine Ausbuchtung 222 aufweist, in die der keilförmige Abschnitt 216 eingreifen kann und an die sich bevorzugt ein Schlitz anschließt, der sich in Längsrichtung L der Blutlanzettenvorrichtung 201 bzw. der Lanzettenaufnahme-einrichtung 218 erstreckt. Innerhalb der Lanzettenaufnahmeeinrichtung 218 ist ein Lanzettenelement 223 reibschlüssig fixiert.

Mit Hilfe des stabförmigen Elementes 225, an dem der keilförmige Abschnitt 216 angeordnet ist, ist dieser in Längsrichtung L verschiebbar. Durch eine Längsverschiebung des keilförmigen Abschnitts 216 ist dieser in den Innenraum 226 der Lanzettenaufnahmeeinrichtung 218 einbringbar. Eine Längsverschiebung des keilförmigen Abschnittes 216 bedeutet eine Längsverschiebung der Fixier- und/oder Ausgabeeinrichtung 214, wodurch das weitere Federelement 228 kraftbeaufschlagt wird. Durch das Federelement 228 wir somit eine Kraft auf die Fixier- und/oder Ausgabeeinrichtung 214 aufgebracht, durch die eine Rückverstellung der Fixier- und/oder Ausgabeeinrichtung 214 in eine Ausgangsposition überführbar ist. In dieser Ausgangsposition liegt ein entkoppelter Zustand vor und der keilförmige Abschnitt 216 bewirkt keine Verformung der Lanzettenaufnahmeeinrichtung 218..

Der Anschlag 233 liegt mit seiner Unterseite bevorzugt flächig an einer Innenwandung 240 an, wodurch mittels dem Handbetätigungselement 206 auf die Fixier- und/oder Ausgabeeinrichtung 214 in Breitenrichtung B übertragene Kräfte am Gehäuse 202 bzw. der Innenwandung 240 abgestützt werden. Ferner bildet die Innenwandung 240 eine Gleitfläche, auf der der Anschlag 233 verschiebbar ist.

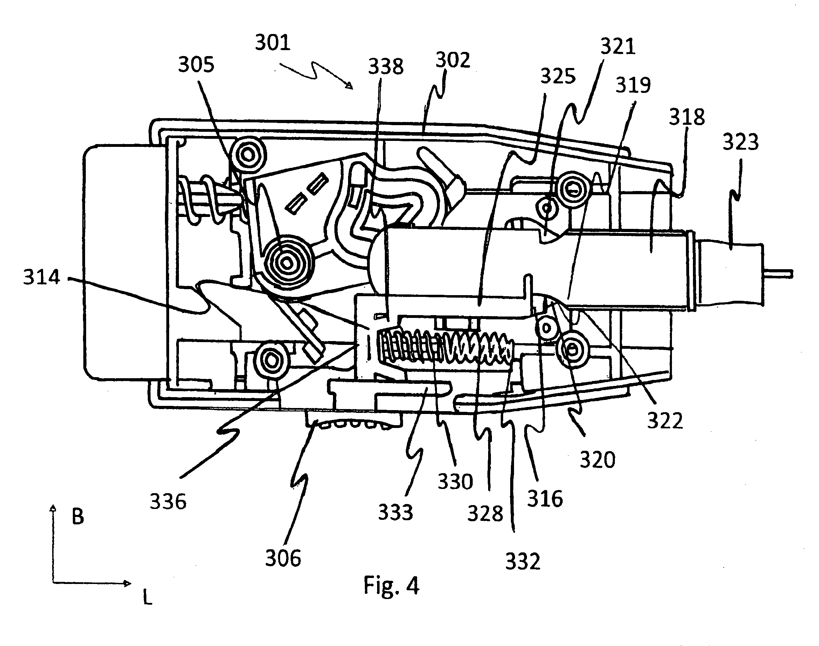

In Fig. 4 ist eine Blutlanzettenvorrichtung 301 dargestellt, in welcher die Fixer- und/oder Ausgabeeinrichtung 314 teilweise verschoben ist. In der dargestellten Position fährt der keilförmige Abschnitt 316 der Fixier- und/oder Ausgabeeinrichtung 314 in einer rückseitig ausgebildeten Ausbuchtung 322 der Lanzettenaufnahmeeinrichtung 318 ein und beginnt das Lanzettenelement 323 rückseitig anzuschieben. Zugleich wird durch den Bereich 319 in der Ausbuchtung 322 das keilförmige Element 316 gezielt auf den Schlitz (nicht dargestellt) zugeführt und es findet eine anfängliche Aufspreizung der Lanzettenaufnahmeeinrichtung 318 statt. Somit berührt die Fixier- und/oder Ausgabeeinrichtung 314 während der hier dargestellten Schiebebewegung zunächst die Rückseite bzw. den Boden des Lanzettenelementes 323 und drückt dieses mit dem Frontbereich 317a während der Verschiebebewegungen in Längsrichtung L aus der Blutlanzettenvorrichtung 301 bzw. der Lanzettenaufnahmeeinrichtung 318 heraus.

Anschließend wird zusätzlich bzw. weiterhin die seitlich geschlitzte Lanzettenaufnahmeeinrichtung 318 mit Hilfe des Schiebelementes bzw. der Fixier- und/oder Aufnahmeeinrichtung 314 aufgedrückt und die Klemmung des Lanzettenelementes 323 über den Umfang der Lanzettenaufnahmeeinrichtung 318 aufgehoben. Ferner ist das weitere Federelement 328 in dieser Darstellung teilweise komprimiert.

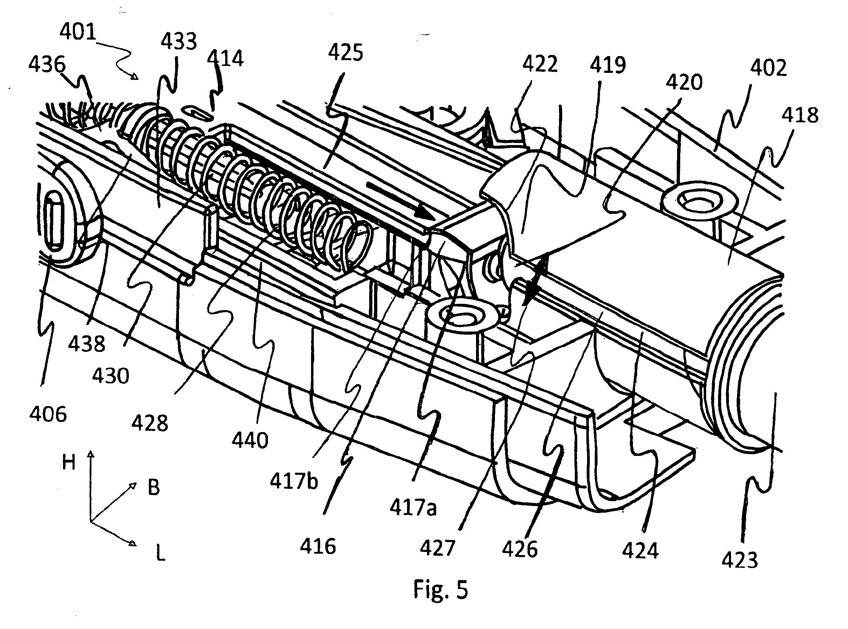

In der Fig. 5 ist eine offene dreidimensionale Darstellung der Blutlanzettenvorrichtung 401 gezeigt. Mittels dem Doppelpfeil 427 ist eine Verformungs- bzw. Aufdehnungsbewegung der Blutlanzettenaufnahmeeinrichtung 418 während eines Einschiebevorganges dargestellt. Es ist ersichtlich, dass der keilförmige Abschnitt 416 in die Ausbuchtung 422 eingreift und sich zumindest teilweise in den Innenraum 426 der Lanzettenaufnahmeeinrichtung 418 erstreckt. Durch die Ausbuchtung 422 findet eine definierte Zuführung des keilförmigen Abschnittes 416 in den Schlitz 424 statt, wobei bevorzugt bereits während dieser Einführbewegung ein Ausschieben des Lanzettenelementes 423 und ein Aufspreizen der Lanzettenaufnahmeeinrichtung 418 erfolgt.

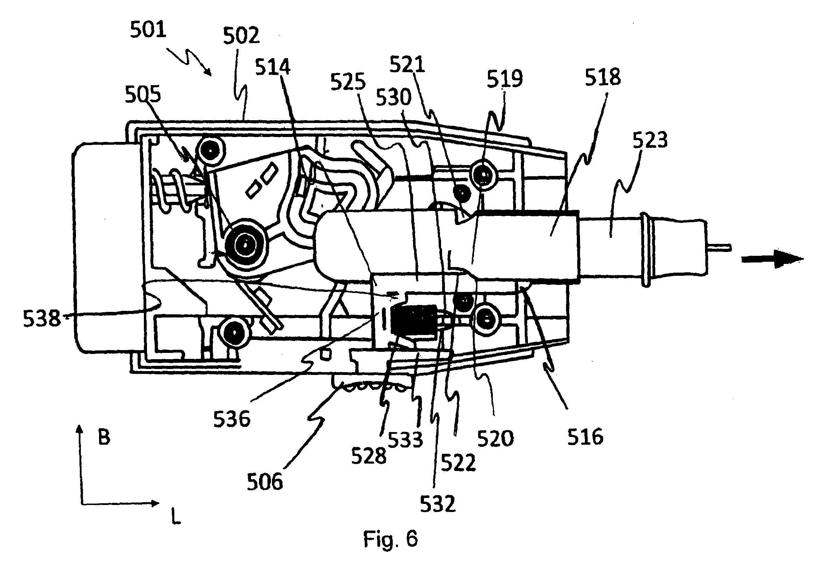

In Fig. 6 ist eine Blutlanzettenvorrichtung 501 gezeigt, in der die Aufhebung der Klemmung und das Aufdrücken der Lanzettenaufnahmeeinrichtung 518 mit Hilfe des Schiebeelementes bzw. der Fixier- und/oder Ausgabeeinrichtung 514 deutlich dargestellt ist. Hierbei findet sich die Fixier- und/oder Ausgabeeinrichtung 514 in einem Endanschlag, an dem der Anschlag 533 mit dem Grundkörper 502 in Kontakt steht. Es findet also ein Auswurf des Lanzettenelementes 523 mittels der Fixier- und/oder Ausgabeeinrichtung 514 statt. Das weitere Federelement 528, das als Druckfeder ausgebildet ist, ist in dieser Endanschlagsposition vollständig komprimiert. Der keilförmige Abschnitt 516 der Fixier- und/oder Ausgabeeinrichtung 514 befindet sich nun zumindest in Längsrichtung L vollständig innerhalb des Schlitzes (nicht gezeigt). Der Schlitz bzw. die Lanzettenaufnahmeeinrichtung 518 ist in dieser Position derart geweitet, dass das Lanzettenelement 523 bevorzugt auch ohne weitere Kraftbeaufschlagung durch die Fixier- und/oder Ausgabeeinrichtung 514 aus der Lanzettenaufnahmeeinrichtung 518 herausfallen kann, ohne dass das Lanzettenelement 523 mit der Hand angefasst werden muss. Dieses Herausfallen wird bevorzugt durch ein Anschieben der Fixier- und/oder Ausgabeeinrichtung 514 von hinten bewirkt oder zumindest unterstützt, wobei die Fixier- und/oder Ausgabeeinrichtung 514 mit dem keilförmigen Abschnitt 516 zugleich eine Aufweitung der Lanzettenaufnahmeeinrichtung 518 bzw. des Lanzettenhalters und ein Herausschieben des Lanzettenelements 523 aus der Lanzettenaufnahmeeinrichtung 518 bewirkt.

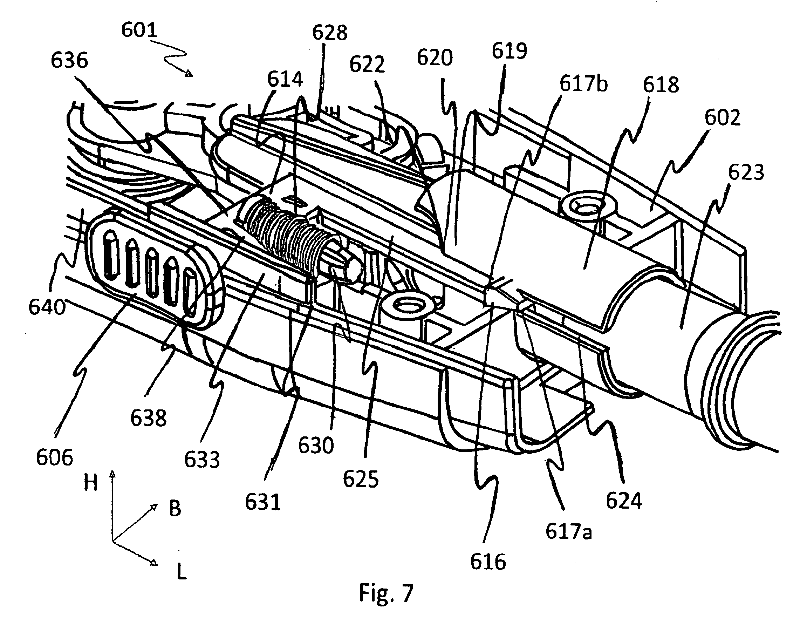

In Fig. 7 ist eine weitere offene dreidimensionale Darstellung der Blutlanzettenvorrichtung 601 dargestellt. Aus dieser Figur geht hervor, dass die Fixier- und/oder Ausgabeeinrichtung 614 mittels dem keilförmigen Abschnitt 616 und dem stabförmigen Element 625 zumindest teilweise mit der Lanzettenaufnahmeeinrichtung 618 in Kontakt steht und diese aufweitet. Ferner ist ersichtlich, dass der Frontbereich 617a des keilförmigen Abschnittes 616, der sich in den Innenraum 624 der Lanzettenaufnahmeeinrichtung 618 erstreckt ein Ausschieben des Lanzettenelementes 623 bewirkt.

Weiterhin ist ersichtlich, dass sich das weitere Federelement 628 in einem komprimierten Zustand befindet. Durch das Mittel 630 ist hierbei eine Ausrichtung bzw. Stabilisierung des Federelementes 628 gegeben. Der Anschlag 633 steht hierbei mit einem Anschlag 631, der am Grundkörper 602 ausgebildet ist, formschlüssig in Verbindung.

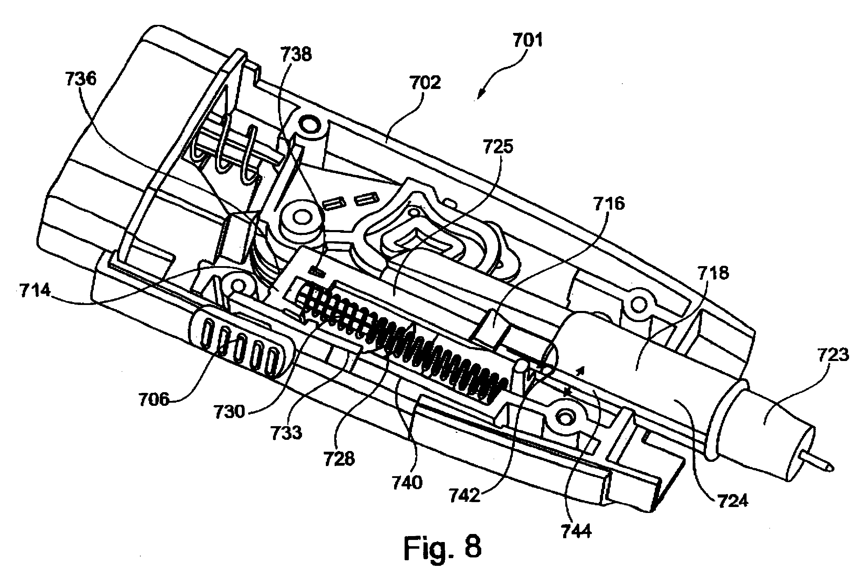

In Fig. 8 ist eine weitere perspektivische Darstellung wiedergegeben, in der sich die Fixier- und/oder Ausgabeeinrichtung 714 in einer gerade begonnen Schiebebewegung, wie es durch den Pfeil 742 wiedergegeben wird, befindet. Mittels des Doppelpfeils 744 wird gezeigt, dass die Lanzettenaufnahmeeinrichtung 718 gerade beginnt, sich aufzuweiten.

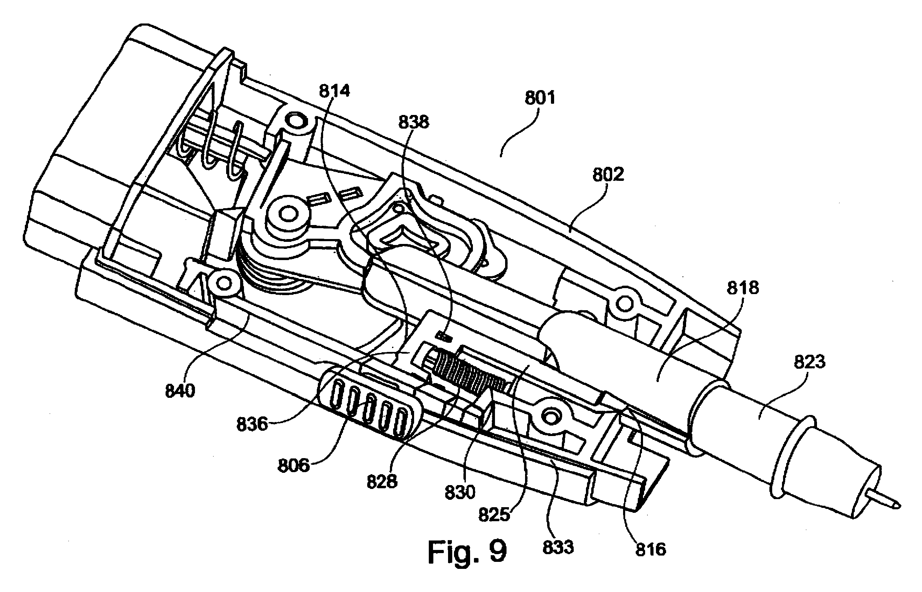

In Fig. 9 ist die Fixier- und/oder Ausgabeeinrichtung 814 mit dem Handbestätigungselement 806 bereits in der vordersten Stellung und hat hierbei mittels des keilförmigen Abschnitts 816 die Lanzettenaufnahmeeinrichtung 818 vollständig aufgeweitet. Eine Entnahme des Lanzettenelementes 823 ist somit durch die gelöste Klemmverbindung möglich. Hierzu drückt die Fixier- und/oder Ausgabeeinrichtung 814 das Lanzettenelement 823 bevorzugt bis zur Hälfte aus der Lanzettenaufnahmeeinrichtung 818 heraus während zeitgleich die Lanzettenaufnahmeeinrichtung aufgeweitet wird, um den Auswurf des Lanzettenelements 823 zu ermöglichen. Anschließend findet eine Rückbewegung der Fixier- und/oder Ausgabeeinrichtung 814 in eine Ausgangsposition statt. Dies erfolgt mittels der Druckfeder bzw. des weiteren Federelementes 828.

Sämtliche in den Anmeldungsunterlagen offenbarten Merkmale werden als erfindungswesentlich beansprucht, sofern sie einzeln oder in Kombination gegenüber dem Stand der Technik neu sind.

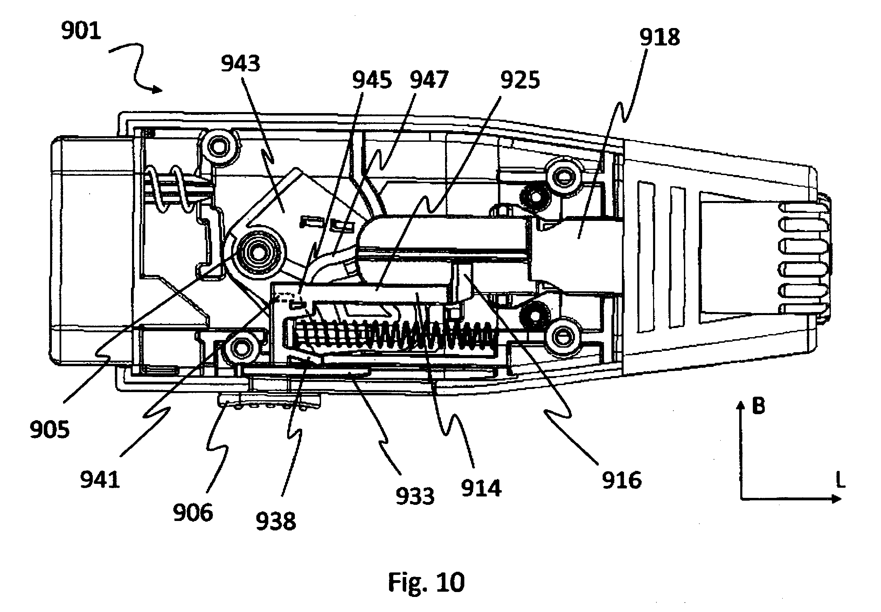

Fig. 10 zeigt einen Zustand der Blutlanzettenvorrichtung 901, in dem das Federelement 905, das mit dem Steuerkurvenelement 943 gekoppelt ist, gespannt ist, d.h. eine Stechbewegung der Blutlanzettenvorrichtung 901 auslösbar ist. Das Steuerkurvenelement 943 weist hierbei eine Kurvenbahn 947 auf, in der die Lanzettenaufnahmeeinrichtung 918 mittels eines Zapfenelementes (nicht gezeigt) geführt bzw. gleitend gelagert ist. Eine Schwenkbewegung des Steuerkurvenelementes 943 führt somit zu einer linearen Bewegung der Lanzettenaufnahmeeinrichtung 918 in Längsrichtung L. In dem in Fig. 10 gezeigten Zustand erfolgt durch das Zusammenwirken der Wandung 945, welche zumindest teilweise die Kurvenbahn 947 bildet, mit einem Zapfenelement 941 (mit Strichlinien dargestellt, da es sich in dieser Darstellung auf der Unterseite der Fixier- und/oder Ausgabeeinrichtung befindet), das an der Fixier- und/oder Ausgabeeinrichtung 914 angeordnet ist, eine Arretierung bzw. eine Begrenzung der Verschiebebewegung der Fixier- und/oder Ausgabeeinrichtung 914. Die Begrenzung der Verschiebebewegung bzw. die Arretierung der Fixier- und/oder Ausgabeeinrichtung 914 erfolgt mittels Formschluss, d.h. die Wandung 945 befindet sich in dem dargestellten Zustand in Längsrichtung L, d.h. in der Ausschieberichtung, vor dem Zapfenelement 941 und bildet daher einen Anschlag für das Zapfenelement 941. Durch ein Auslösen der Stechbewegung erfolgt aufgrund der im Federelement 905 gespeicherten Energie eine Auslenkung des Steuerkurvenelements 943, wodurch sich dieses aus dem Verschiebebereich der Fixier- und/oder Ausgabeeinrichtung 914 heraus bewegt und dadurch eine Verschiebung der Fixier- und/oder Ausgabeeinrichtung 914 zulässt. Die Blutlanzettenvorrichtung 901 ist daher derart gestaltet, dass lediglich in einem ungespannten bzw. zumindest teilweise entspannten Zustand des Federelements 905 eine Verschiebung der Fixier- und/oder Ausgabeeinrichtung 914 möglich ist.

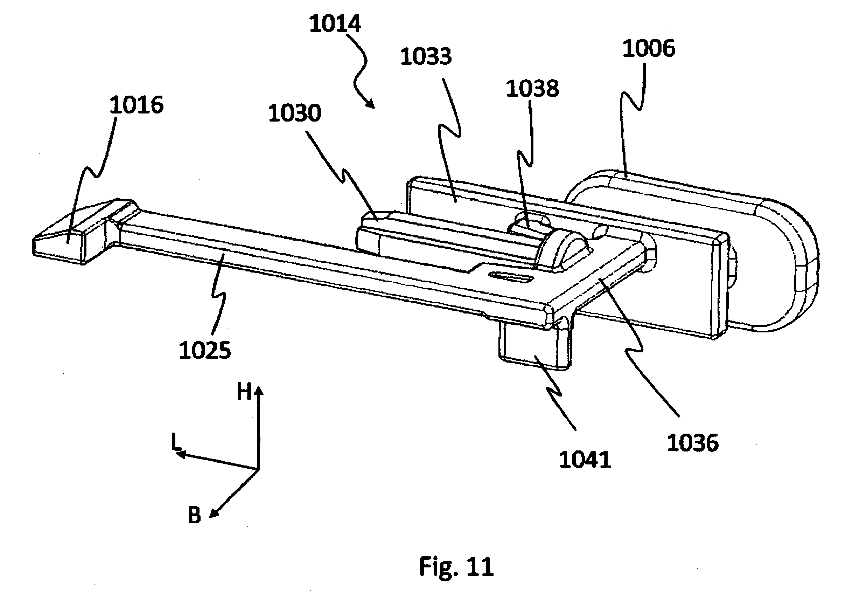

Fig. 11 zeigt eine dreidimensionale Darstellung der Fixier- und/oder Ausgabeeinrichtung 1014, die bevorzugt einstückig und aus Kunststoff gefertigt ist. Die Fixier- und/oder Ausgabeeinrichtung 1014 weist ein Handbetätigungselement 1006, einen Anschlag 1033, ein Trägerelement 1036, an dem ein Mittel 1030 und zwei Versteifungselemente 1038 angeordnet sind, und ein stabförmiges Element 1025, an dem der keilförmige Abschnitt 1016 angeordnet ist, auf. Ferner erstreckt sich im Bereich der Verbindungsstelle bzw. im Übergangsbereich zwischen dem Trägerelement 1036 und dem stabförmigen Element 1025 ein Zapfenelement 1041 im Wesentlichen in Höhenrichtung H und in Breitenrichtung B. Es ist hierbei jedoch ebenfalls denkbar, dass das Zapfenelement 1041 in einer abweichenden Form ausgebildet ist, sofern ein Zusammenwirken des Zapfenelements 1041 mit der Wandung 947 (vgl. Fig. 10) möglich ist.

### Bezugszeichenliste

- 1, 101, 201, 301, 401, 501, 601, 701, 801, 901: Blutlanzettenvorrichtung
- 2, 102, 202, 302, 402, 502, 602, 702,802: Grundkörper
- 4: Betätigungselement
- 105, 305, 505, 905: Federelement
- 6, 106, 206, 306, 406, 506, 606, 706, 806, 906, 1006: Handbetätigungselement
- 8: Kappe
- 10: Stellrad
- 12: Markierung
- 114, 214, 314, 414, 514, 614, 714, 814, 914, 1014: Fixier- und/oder Ausgabeeinrichtung
- 116, 216, 316, 416, 516, 616, 716, 816, 916, 1016: keilförmiger Abschnitt
- 217a, 417a, 617a: schlitzartiger Frontbereich
- 217b, 417b, 617b: Endbereich
- 118, 218, 318, 418, 518, 618, 718, 818, 918: Lanzettenaufnahmeeinrichtung
- 119, 219, 319, 419, 519, 619: Abschnitt der Lanzettenaufnahmeeinrichtung
- 120,220,320,420,520,620: Ausnehmung
- 121,321,521: zweite Ausnehmung
- 122, 222, 322, 422, 522, 622: Ausbuchtung
- 123, 223, 323, 423, 523, 623, 723, 823: Lanzettenelement
- 224, 424, 624, 724: Schlitz
- 125, 225, 325, 425, 525, 625, 725, 825, 925, 1025: stabförmiges Element
- 226, 426: Innenraum d. Lanzettenaufnahmeeinrichtung
- 427: Doppelpfeil
- 128, 228, 328, 428, 528, 628, 728, 828: weiteres Federelement
- 130, 230, 330, 430, 530, 630, 730, 830, 1030: Mittel der Fixier- u./o. Ausgabeeinrichtung
- 631: Anschlag am Grundkörper
- 132, 332, 532: am Grundkörper angebrachte Mittel
- 133, 233, 333, 433, 533, 633, 733, 833, 933, 1033: Anschlag
- 136, 236, 336, 436, 536, 636, 736, 836, 1036: Trägerelement
- 138, 238, 338, 438, 538, 638, 738, 838, 938, 1038: Versteifungselemente
- 240, 440, 640, 740, 840: Innenwandung
- 941, 1041: Zapfenelement
- 742: Pfeil
- 943: Steuerkurvenelement
- 744: Doppelpfeil
- 945: Wandung
- 947: Kurvenbahn
- L: Längsrichtung
- B: Breitenrichtung
- H: Höhenrichtung

## Patentansprüche

1. Blutlanzettenvorrichtung mit einem Grundkörper (2, 102, 202, 302, 402, 502, 602, 702,802) zum Anordnen einer Vielzahl an Vorrichtungskomponenten umfassend eine Lanzettenaufnahmeeinrichtung (118, 218, 318, 418, 518, 618, 718, 818, 918) zum Koppeln der Blutlanzettenvorrichtung mit einem Lanzettenelement (123, 223, 323, 423, 523, 623, 723, 823),
mindestens ein Federelement (105, 305, 505, 905) zum Federkraftbeaufschlagen der Lanzettenaufnahmeeinrichtung und
ein Betätigungselement (4) zum Auslösen einer Bewegung der Lanzettenaufnahmeeinrichtung,
wobei mittels einer Fixier- und/oder Ausgabeeinrichtung (114, 214, 314, 414, 514, 614, 714, 814, 914, 1014) die Kopplung zwischen der Lanzettenaufnahmeeinrichtung und dem Lanzettenelement veränderbar ist,
wobei
mittels einer Bewegung der Fixier- und/oder Ausgabeeinrichtung (114, 214, 314, 414, 514, 614, 714, 814, 914, 1014) in Längsrichtung (L) der Lanzettenaufnahmeeinrichtung (118, 218, 318, 418, 518, 618, 718, 818, 918) und mittels eines Eingreifens der
Fixier- und/oder Ausgabeeinrichtung in mindestens eine Ausnehmung der Lanzettenaufnahmeeinrichtung die Lanzettenaufnahmeeinrichtung verformbar ist, **dadurch gekennzeichnet,dass**
an der Fixier- und/oder Ausgabeeinrichtung (114, 214, 314, 414, 514, 614, 714, 814, 914, 1014) ein Handbetätigungselement (6, 106, 206, 306, 406, 506, 606, 706, 806, 906, 1006) direkt angeordnet ist.

2. Blutlanzettenvorrichtung nach Anspruch 1,
**dadurch gekennzeichnet, dass**
ein Abschnitt (116, 216, 316, 416, 516, 616, 716, 816, 916, 1016) der Fixier- und/oder Ausgabeeinrichtung (114, 214, 314, 414, 514, 614, 714, 814, 914, 1014) keilförmig gestaltet ist und durch ein Zusammenwirken des keilförmigen Abschnitts mit einem Abschnitt (119, 219, 319, 419, 519, 619) der Lanzettenaufnahmeeinrichtung eine Veränderung eines Querschnitts der Lanzettenaufnahmeeinrichtung durchführbar ist.

3. Blutlanzettenvorrichtung nach Anspruch 2,
**dadurch gekennzeichnet, dass**
die Lanzettenaufnahmeeinrichtung (118, 218, 318, 418, 518, 618, 718, 818, 918) zumindest abschnittsweise rohrförmig ausgebildet ist und der Abschnitt (119, 219, 319, 419, 519, 619) der Lanzettenaufnahmeeinrichtung im Bereich der mindestens einen Ausnehmung (120, 220, 320, 420, 520, 620), bevorzugt in der mindestens einen Ausnehmung, ausgebildet ist, wobei bevorzugt mindestens zwei Ausnehmungen (121, 321, 521) in der Lanzettenaufnahmeeinrichtung vorgesehen sind, die gegenüber von einander angeordnet sind, und sich zumindest eine Ausnehmung zumindest abschnittsweise in Längsrichtung (L) erstreckt und schlitzartig ausgebildet ist.

4. Blutlanzettenvorrichtung nach Anspruch 3,
**dadurch gekennzeichnet,dass**
der keilförmige Abschnitt (116, 216, 316, 416, 516, 616, 716, 816, 916, 1016) der Fixier- und/oder Ausgabeeinrichtung (114, 214, 314, 414, 514, 614, 714, 814, 914, 1014) in eine Ausbuchtung (122, 222, 322, 422, 522, 622) der Ausnehmung des Lanzettenaufnahmeeinrichtung einbringbar ist und der Schlitz (224, 424, 624, 724) eine weitere Erstreckung der Ausbuchtung (122, 222, 322, 422, 522, 622) darstellt.

5. Blutlanzettenvorrichtung nach einem der Ansprüche 3 bis 4,
**dadurch gekennzeichnet,dass**
der keilförmige Abschnitt (116, 216, 316, 416, 516, 616, 716, 816, 916, 1016) der Fixier- und/oder Ausgabeeinrichtung (114, 214, 314, 414, 514, 614, 714, 814, 914, 1014) zumindest zeitweise und abschnittsweise mit dem Abschnitt der Lanzettenaufnahmeeinrichtung (118, 218, 318, 418, 518, 618, 718, 818, 918) in Kontakt bringbar ist und eine Kraft durch die Fixier- und/oder Ausgabeeinrichtung manuell auf das Lanzettenelement (123, 223, 323, 423, 523, 623, 723, 823) übertragbar ist, wobei durch das Aufbringen der Kraft zeitgleich das Lanzettenelement zumindest teilweise in der Ausgaberichtung (L) bewegbar und der Schlitz (224, 424, 624, 724) veränderbar ist.

6. Blutlanzettenvorrichtung nach einem der Ansprüche 3 bis 5,
**dadurch gekennzeichnet,dass**
der keilförmige Abschnitt (116, 216, 316, 416, 516, 616, 716, 816, 916, 1016) einen spitzenartigen Frontbereich (217a, 417a, 617a) aufweist, der zumindest abschnittsweise in den Schlitz (224, 424, 624, 724) einführbar und zumindest abschnittsweise in einen das Lanzettenelement (123, 223, 323, 423, 523, 623, 723, 823) aufnehmenden Innenraum (226, 426) der Lanzettenaufnahmeeinrichtung (118, 218, 318, 418, 518, 618, 718, 818, 918) einführbar ist.

7. Blutlanzettenvorrichtung nach einem der Ansprüche 2 bis 6,
**dadurch gekennzeichnet,dass**
die Fixier- und/oder Ausgabeeinrichtung (114, 214, 314, 414, 514, 614, 714, 814, 914, 1014) ein stabförmiges Element (125, 225, 325, 425, 525, 625, 725, 825, 925, 1025) aufweist, an dessen Ende der keilförmige Abschnitt (116, 216, 316, 416, 516, 616, 716, 816, 916, 1016), bevorzugt seitlich versetzt, angeordnet ist.

8. Blutlanzettenvorrichtung nach Anspruch 7,
**dadurch gekennzeichnet,dass**
der keilförmige Abschnitt (116, 216, 316, 416, 516, 616, 716, 816, 916, 1016) eine Unterseite aufweist, die mit einer Unterseite des stabförmigen Elements (125, 225, 325, 425, 525, 625, 725, 825, 925, 1025) in einer gemeinsamen Ebene angeordnet ist.

9. Blutlanzettenvorrichtung nach einem der Ansprüche 7 oder 8,
**dadurch gekennzeichnet, dass**
der keilförmige Abschnitt (116, 216, 316, 416, 516, 616, 716, 816, 916, 1016) neben dem spitzenartigen Frontbereich einen Endbereich (217b, 417b, 617b) aufweist, wobei die Querschnittshöhe (H1) des spitzenartigen Frontbereichs (217a, 417a, 617a) bevorzugt geringer ist als die Querschnittshöhe (H2) des stabförmigen Elements (125, 225, 325, 425, 525, 625, 725, 825, 925, 1025) und die Querschnittshöhe (H3) des Endbereichs (217b, 417b, 617b) bevorzugt höher ist als die Querschnittshöhe (H2) des stabförmigen Elements.

10. Blutlanzettenvorrichtung nach einem der vorangegangenen Ansprüche,
**dadurch gekennzeichnet, dass**
das die Bewegung der Fixier- und/oder Ausgabeeinrichtung (114, 214, 314, 414, 514, 614, 714, 814, 914, 1014) durch mindestens ein weiteres Federelement (128, 228, 328, 428, 528, 628, 728, 828) mit dem Grundkörper (2, 102, 202, 302, 402, 502, 602, 702,802) gekoppelt ist, das Federelement durch an der Fixier- und/oder Ausgabeeinrichtung und/oder dem Grundkörper vorgesehene Mittel (132, 332, 532) geführt ist und die Fixier- und/oder Ausgabeeinrichtung mittels dem Federelement entgegen der Ausgaberichtung (L) bewegbar ist.

11. Blutlanzettenvorrichtung nach einem der vorhergehenden Ansprüche,
**dadurch gekennzeichnet,dass**
an der Fixier- und/oder Ausgabeeinrichtung (114, 214, 314, 414, 514, 614, 714, 814, 914, 1014) ein Anschlag (133, 233, 333, 433, 533, 633, 733, 833, 933, 1033) vorgesehen ist, der derart mit dem Grundkörper zusammenwirkt, dass der Verschiebeweg der Fixier- und/oder Ausgabeeinrichtung begrenzt ist.

12. Blutlanzettenvorrichtung nach Anspruch 11,
**dadurch gekennzeichnet,dass**
auf einer Seite des Anschlages (133, 233, 333, 433, 533, 633, 733, 833, 933, 1033) der Fixier- und/oder Ausgabeeinrichtung (114, 214, 314, 414, 514, 614, 714, 814, 914, 1014) ein Handbetätigungselement (6, 106, 206, 306, 406, 506, 606, 706, 806, 906, 1006) vorgesehen ist.

13. Blutlanzettenvorrichtung nach Anspruch 10,
**dadurch gekennzeichnet,dass**
die Fixier- und/oder Ausgabeeinrichtung (114, 214, 314, 414, 514, 614, 714, 814, 914, 1014) ein sich im Wesentlichen rechtwinklig zur Längsrichtung (L) erstreckendes Trägerelement (136, 236, 336, 436, 536, 636, 736, 836, 1036) aufweist, an dem im Wesentlichen rechtwinklig das Mittel (130, 230, 330, 430, 530, 630, 730, 830, 1030) zum Führen des weiteren Federelements (128, 228, 328, 428, 528, 628, 728, 828), das stabförmige Element (125, 225, 325, 425, 525, 625, 725, 825, 925, 1025) und der Anschlag (133, 233, 333, 433, 533, 633, 733, 833, 933, 1033) vorgesehen sind, wobei bevorzugt zwischen dem Anschlag und dem Mittel zum Führen des weiteren Federelements und/oder zwischen dem stabförmigen Element (125, 225, 325, 425, 525, 625, 725, 825, 925, 1025) und dem Mittel zum Führen des weiteren Federelements Versteifungselemente (138, 238, 338, 438, 538, 638, 738, 838, 938, 1038) vorgesehen sind.

14. Blutlanzettenvorrichtung nach einem der Ansprüche 2 bis 13,
**dadurch gekennzeichnet, dass**
zumindest der keilförmige Abschnitt (116, 216, 316, 416, 516, 616, 716, 816, 916, 1016) einstückig mit der übrigen Fixier- und/oder Ausgabeeinrichtung (114, 214, 314, 414, 514, 614, 714, 814, 914, 1014), insbesondere mit dem stabförmigen Element (125, 225, 325, 425, 525, 625, 725, 825, 925, 1025), hergestellt ist.

15. Blutlanzettenvorrichtung nach einem der vorhergehenden Ansprüche,
**dadurch gekennzeichnet, dass**
der Grundkörper (2, 102, 202, 302, 402, 502, 602, 702,802) zumindest eine ebene Innenwandung (240, 440, 640, 740, 840) zum definierten verschieben der Fixier- und/oder Ausgabeeinrichtung (114, 214, 314, 414, 514, 614, 714, 814, 914, 1014) aufweist.

## Claims

1. A blood lancet apparatus with a base member (2, 102, 202, 302, 402, 502, 602, 702, 802) for the arrangement of a plurality of apparatus components comprising a lancet-receiving device (118, 218, 318, 418, 518, 618, 718, 818, 918) for coupling the blood lancet apparatus to a lancet element (123, 223, 323, 423, 523, 623, 723, 823), at least one spring element (105, 305, 505, 905) for acting upon the lancet-receiving device with spring force and an actuating element (4) for initiating a movement of the lancet-receiving device, wherein the coupling between the lancet-receiving device and the lancet element is capable of being altered by means of a fixing and/or dispensing device (114, 214, 314, 414, 514, 614, 714, 814, 914, 1014), wherein the lancet-receiving device (118, 218, 318, 418, 518, 618, 718, 818, 918) is capable of being deformed by means of a movement of the fixing and/or dispensing device (114, 214, 314, 414, 514, 614, 714, 814, 914, 1014) in the longitudinal direction (L) of the lancet-receiving device (118, 218, 318, 418, 518, 618, 718, 818, 918)and by means of an engagement of the fixing and/or dispensing device in at least one recess in the lancet-receiving device, **characterized in that**
a manual actuation element (6, 106, 206, 306, 406, 506, 606, 706, 806, 906, 1006) is directly arranged on the fixing and/or dispensing device (114, 214, 314, 514,614, 714, 814, 914, 1014).

2. A blood lancet apparatus according to claim 1, **characterized in that** a portion (116, 216, 316, 416, 516, 616, 716, 816, 916, 1016) of the fixing and/or dispensing device (114, 214, 314, 414, 514, 614, 714, 814, 914, 1014) is made wedge-shaped, and by means of a co-operation of the wedge-shaped portion with a section (119, 219, 319, 419, 519, 619) of the lancet-receiving device a change in a cross-section of the lancet-receiving device is capable of being carried out.

3. A blood lancet apparatus according to claim 2, **characterized in that** the lancet-receiving device (118, 218, 318, 418, 518, 618, 718, 818, 918) is constructed at least sectionally in the form of a tube and the portion (119, 219, 319, 419, 519, 619) of the lancet-receiving device is formed in the region of the at least one recess (120, 220, 320, 420, 520, 620), preferably in the at least one recess, wherein at least two recesses (121, 321, 521), which are arranged opposite each other, are preferably provided in the lancet-receiving device, and at least one recess extends at least sectionally in the longitudinal direction (L) and is designed in the manner of a slot.

4. A blood lancet apparatus according to claim 3, **characterized in that** the wedge-shaped portion (116, 216, 316, 416, 516, 616, 716, 816, 916, 1016) of the fixing and/or dispensing device (114, 214, 314, 414, 514, 614, 714, 814, 914, 1014) is capable of being introduced into an indentation (122, 222, 322, 422, 522, 622) of the recess of the lancet-receiving device, and the slot (224, 424, 624, 724) represents a further extension of the indentation (122, 222, 322, 422, 522, 622).

5. A blood lancet apparatus according to any one of claims 3 to 4, **characterized in that** the wedge-shaped portion (116, 216, 316, 416, 516, 616, 716, 816, 916, 1016) of the fixing and/or dispensing device (114, 214, 314, 414, 514, 614, 714, 814, 914, 1014) is capable of being brought into contact at least for a time and sectionally with the portion of the lancet-receiving device (118, 218, 318, 418, 518, 618, 718, 818, 918), and a force is capable of being transmitted manually through the fixing and/or dispensing device to the lancet element (123, 223, 323, 423, 523, 623, 723, 823), wherein as a result of the application of the force it is possible at the same time for the lancet element to be moved at least in part in the dispensing direction (L) and for the slot (224, 424, 624, 724) to be altered.

6. A blood lancet apparatus according to any one of claims 3 to 5, **characterized in that** the wedge-shaped portion (116, 216, 316, 416, 516, 616, 716, 816, 916, 1016) has a pointed front region (217a, 417a, 617a) which is capable of being introduced at least sectionally into the slot (224, 424, 624, 724), and it is capable of being introduced at least sectionally into an inner space (226, 426) of the lancet-receiving device (118, 218, 318, 418, 518, 618, 718, 818, 918) which receives the lancet element (123, 223, 323, 423, 523, 623, 723, 823).

7. A blood lancet apparatus according to any one of claims 2 to 6, **characterized in that** the fixing and/or dispensing device (114, 214, 314, 414, 514, 614, 714, 814, 914, 1014) has a rod-shaped element (125, 225, 325, 425, 525, 625, 725, 825, 925, 1025), on the end of which the wedge-shaped portion (116, 216, 316, 416, 516, 616, 716, 816, 916, 1016) is arranged, preferably offset laterally.

8. A blood lancet apparatus according to claim 7, **characterized in that** the wedge-shaped portion (116, 216, 316, 416, 516, 616, 716, 816, 916, 1016) has an underside which is situated in a common plane with an underside of the rod-shaped element (125, 225, 325, 425, 525, 625, 725, 825, 925, 1025).

9. A blood lancet apparatus according to one of claims 7 or 8, **characterized in that** the wedge-shaped portion (116, 216, 316, 416, 516, 616, 716, 816, 916, 1016) has an end region (217b, 417b, 617b) in addition to the pointed front region, wherein the cross-sectional height (H1) of the pointed front region (217a, 417a, 617a) is preferably lower than the cross-sectional height (H2) of the rod-shaped element (125, 225, 325, 425, 525, 625, 725, 825, 925, 1025), and the cross-sectional height (H3) of the end region (217b, 417b, 617b) is preferably higher than the cross-sectional height (H2) of the rod-shaped element

10. A blood lancet apparatus according to any one of the preceding claims, **characterized in that** the movement of the fixing and/or dispensing device (114, 214, 314, 414, 514, 614, 714, 814, 914, 1014) is coupled to the base member (2, 102, 202, 302, 402, 502, 602, 702, 802) by at least one further spring element (128, 228, 328, 428, 528, 628, 728, 828), the spring element is guided by means (132, 332, 532) provided on the fixing and/or dispensing device and/or on the base member and the fixing and/or dispensing device is capable of being moved in a direction opposed to the dispensing direction (L) by means of the spring element.

11. A blood lancet apparatus according to any one of the preceding claims, **characterized in that** the fixing and/or dispensing device (114, 214, 314, 414, 514, 614, 714, 814, 914, 1014) has provided on it a stop (133, 233, 333, 433, 533, 633, 733, 833, 933, 1033) which co-operates with the base member in such a way that the displacement path of the fixing and/or dispensing device is limited.

12. A blood lancet apparatus according to claim 11, **characterized in that** a manual actuation element (6, 106, 206, 306, 406, 506, 606, 706, 806, 906, 1006) is provided on one side of the stop (133, 233, 333, 433, 533, 633, 733, 833, 933, 1033) of the fixing and/or dispensing device (114, 214, 314, 414, 514, 614, 714, 814, 914, 1014).

13. A blood lancet apparatus according to claim 10, **characterized in that** the fixing and/or dispensing device (114, 214, 314, 414, 514, 614, 714, 814, 914, 1014) has a carrier element (136, 236, 336, 436, 536, 636, 736, 836, 1036) which extends substantially at a right angle to the longitudinal direction (L) and on which the means (130, 230, 330, 430, 530, 630, 730, 830, 1030) for guiding the further spring element (128, 228, 328, 428, 528, 628, 728, 828), the rod-shaped element (125, 225, 325, 425, 525, 625, 725, 825, 925, 1025) and the stop (133, 233, 333, 433, 533, 633, 733, 833, 933, 1033) are provided substantially at a right angle, wherein reinforcement elements (138, 238, 338, 438, 538, 638, 738, 838, 938, 1038) are preferably provided between the stop and the means for guiding the further spring element and/or between the rod-shaped element (125. 225, 325, 425, 525, 625, 725, 825, 925, 1025) and the means for guiding the further spring element.

14. A blood lancet apparatus according to any one of claims 2 to 13, **characterized in that** at least the wedge-shaped portion (116, 216, 316, 416, 516, 616, 716, 816, 916, 1016) is produced in one piece with the remainder of the fixing and/or dispensing device (114, 214, 314, 414, 514, 614, 714, 814, 914, 1014), in particular with the rod-shaped element (125, 225, 325, 425, 525, 625, 725, 825, 925, 1025).

15. A blood lancet apparatus according to any one of the preceding claims, **characterized in that** the base member (2, 102, 202, 302, 402, 502, 602, 702, 802) has at least one flat inner wall (240, 440, 640, 740, 840) for displacing the fixing and/or dispensing device (114, 214, 314, 414, 514, 614, 714, 814, 914, 1014) in a defined manner.

## Revendications

1. Dispositif de lancette de sang, avec un corps de base (2, 102, 202, 302, 402, 502, 602, 702, 802) pour agencer une pluralité de composants du dispositif comprenant
un dispositif porte-lancette (118, 218, 318, 418, 518, 618, 718, 818, 918) pour coupler le dispositif de lancette de sang avec un élément de lancette (123, 223, 323, 423, 523, 623, 723, 823),
au moins un élément de ressort (105, 305, 505, 905) pour appliquer une force de ressort sur le dispositif porte-lancette et
un élément d'actionnement (4) pour déclencher un mouvement du dispositif porte-lancette,
dans lequel le couplage entre le dispositif porte-lancette et l'élément de lancette peut être modifié au moyen d'un dispositif de fixation et/ou d'éjection (114, 214, 314, 414, 514, 614, 714, 814, 914, 1014),
dans lequel
le dispositif porte-lancette est déformable au moyen d'un mouvement du dispositif de fixation et/ou d'éjection (114, 214, 314, 414, 514, 614, 714, 814, 914, 1014) dans la direction longitudinale (L) du dispositif porte-lancette (118, 218, 318, 418, 518, 618, 718, 818, 918) et au moyen d'un engagement du dispositif de fixation et/ou d'éjection dans au moins un évidement du dispositif porte-lancette,
**caractérisé en ce qu'**un élément d'actionnement manuel (6, 106, 206, 306, 406, 506, 606, 706, 806, 906, 1006) est agencé directement contre le dispositif de fixation et/ou d'éjection (114, 214, 314, 414, 514, 614, 714, 814, 914, 1014).

2. Dispositif de lancette de sang selon la revendication 1, **caractérisé en ce qu'**un tronçon (116, 216, 316, 416, 516, 616, 716, 816, 916, 1016) du dispositif de fixation et/ou d'éjection (114, 214, 314, 414, 514, 614, 714, 814, 914, 1014) est conçu de manière cunéiforme et la coopération entre le tronçon cunéiforme et un tronçon (119, 219, 319, 419, 519, 619) du dispositif porte-lancette permet de faire varier une section transversale du dispositif porte-lancette.

3. Dispositif de lancette de sang selon la revendication 2, **caractérisé en ce que** le dispositif porte-lancette (118, 218, 318, 418, 518, 618, 718, 818, 918) est réalisé, au moins par sections, avec une forme tubulaire, et le tronçon (119, 219, 319, 419, 519, 619) du dispositif porte-lancette est réalisé dans la zone du au moins un évidement (120, 220, 320, 420, 520, 620), de préférence dans le au moins un évidement, de préférence au moins deux évidements (121, 321, 521) étant prévus dans le dispositif porte-lancette, lesquels sont agencés face à face, et au moins un évidement s'étend au moins par sections dans la direction longitudinale (L) et est réalisé en forme de fente.

4. Dispositif de lancette de sang selon la revendication 3, **caractérisé en ce que** le tronçon cunéiforme (116, 216, 316, 416, 516, 616, 716, 816, 916, 1016) du dispositif de fixation et/ou d'éjection (114, 214, 314, 414, 514, 614, 714, 814, 914, 1014) peut être inséré dans un bombement (122, 222, 322, 422, 522, 622) de l'évidement du dispositif porte-lancette et la fente (224, 424, 624, 724) constitue une extension supplémentaire du bombement (122, 222, 322, 422, 522, 622).

5. Dispositif de lancette de sang selon l'une quelconque des revendications 3 à 4, **caractérisé en ce que** le tronçon cunéiforme (116, 216, 316, 416, 516, 616, 716, 816, 916, 1016) du dispositif de fixation et/ou d'éjection (114, 214, 314, 414, 514, 614, 714, 814, 914, 1014) peut être amené en contact, au moins temporairement et par tronçons, avec le tronçon du dispositif porte-lancette (118, 218, 318, 418, 518, 618, 718, 818, 918), et une force peut être manuellement transmise par le dispositif de fixation et/ou d'éjection à l'élément de lancette (123, 223, 323, 423, 523, 623, 723, 823), l'application de la force permettant simultanément de déplacer l'élément de lancette au moins partiellement dans la direction d'éjection (L) et de modifier la fente (224, 424, 624, 724).

6. Dispositif de lancette de sang selon l'une quelconque des revendications 3 à 5, **caractérisé en ce que** le tronçon cunéiforme (116, 216, 316, 416, 516, 616, 716, 816, 916, 1016) présente une zone frontale (217a, 417a, 617a) en pointe, qui peut être engagée au moins par tronçons dans la fente (224, 424, 624, 724) et peut être engagée au moins par tronçons dans un espace intérieur (226, 426) du dispositif porte-lancette (118, 218, 318, 418, 518, 618, 718, 818, 918), lequel espace intérieur reçoit l'élément de lancette (123, 223, 323, 423, 523, 623, 723, 823).

7. Dispositif de lancette de sang selon l'une quelconque des revendications 2 à 6, **caractérisé en ce que** le dispositif de fixation et/ou d'éjection (114, 214, 314, 414, 514, 614, 714, 814, 914, 1014) présente un élément en forme de tige (125, 225, 325, 425, 525, 625, 725, 825, 925, 1025), à l'extrémité duquel est agencé le tronçon cunéiforme (116, 216, 316, 416, 516, 616, 716, 816, 916, 1016), de préférence latéralement décalé.

8. Dispositif de lancette de sang selon la revendication 7, **caractérisé en ce que** le tronçon cunéiforme (116, 216, 316, 416, 516, 616, 716, 816, 916, 1016) présente une face inférieure qui est agencée dans un plan commun avec une face intérieure de l'élément en forme de tige (125, 225, 325, 425, 525, 625, 725, 825, 925, 1025).

9. Dispositif de lancette de sang selon la revendication 7 ou 8, **caractérisé en ce que** le tronçon cunéiforme (116, 216, 316, 416, 516, 616, 716, 816, 916, 1016) présente, outre la zone frontale en pointe, une zone d'extrémité (217b, 417b, 617b), la hauteur transversale (H1) de la zone frontale (217a, 417a, 617a) en pointe étant de préférence inférieure à la hauteur transversale (H2) de l'élément en forme de tige (125, 225, 325, 425, 525, 625, 725, 825, 925, 1025), et la hauteur transversale (H3) de la zone d'extrémité (217b, 417b, 617b) étant de préférence supérieure à la hauteur transversale (H2) de l'élément en forme de tige.

10. Dispositif de lancette de sang selon l'une quelconque des revendications précédentes, **caractérisé en ce que** le mouvement du dispositif de fixation et/ou d'éjection (114, 214, 314, 414, 514, b14, 714, 814, 914, 1014) est couplé au corps de base (2, 102, 202, 302, 402, 502, 602, 702, 802) par au moins un élément de ressort supplémentaire (128, 228, 328, 428, 528, 628, 728, 828), l'élément de ressort est guidé par des moyens (132, 332, 532) prévus contre le dispositif de fixation et/ou d'éjection et/ou le corps de base, et le dispositif de fixation et/ou d'éjection peut être déplacé au moyen de l'élément de ressort dans le sens contraire à la direction d'éjection (L).

11. Dispositif de lancette de sang selon l'une quelconque des revendications précédentes, **caractérisé en ce que** sur le dispositif de fixation et/ou d'éjection (114, 214, 314, 414, 514, 614, 714, 814, 914, 1014) est prévue une butée (133, 233, 333, 433, 533, 633, 733, 833, 933, 1033) qui coopère avec le corps de hase de marnière à limiter la trajectoire de déplacement du dispositif de fixation et/ou d'éjection.

12. Dispositif de lancette de sang selon la revendication 11, **caractérisé en ce qu'**il est prévu un élément d'actionnement manuel (6, 106, 206, 306, 406, 506, 606, 706, 806, 906, 1006) sur un côté de la butée (133, 233, 333, 433, 533, 633, 733, 833, 933, 1033) du dispositif de fixation et/ou d'éjection (114, 214, 314, 414, 514, 614, 714, 814, 914, 1014).

13. Dispositif de lancette de sang selon la revendication 10, **caractérisé en ce que** le dispositif de fixation et/ou d'éjection (114, 214, 314, 414, 514, 614, 714, 814, 914, 1014) présente un élément de support (136, 236, 336, 436, 536, 636, 736, 836, 1036) s'étendant sensiblement perpendiculairement à la direction longitudinale (L), contre lequel sont prévus sensiblement perpendiculairement le moyen (130, 230, 330, 430, 530, 630, 730, 830, 1030) pour guider l'élément de ressort supplémentaire (128, 228, 328, 428, 528, 628, 728, 828), l'élément en forme de tige (125, 225, 325, 425, 525, 625, 725, 825, 925, 1025) et la butée (133, 233, 333, 433, 533, 633, 733, 833, 933, 1033), des éléments de renforcement (138, 238, 338, 438, 538, 638, 738, 838, 938, 1038) étant prévus de préférence entre la butée et le moyen de guidage de l'élément de ressort supplémentaire et/ou entre l'élément en forme de tige (125, 225, 325, 425, 525, 625, 725, 825, 925, 1025) et le moyen de guidage de l'élément de ressort supplémentaire.

14. Dispositif de lancette de sang selon l'une quelconque des revendications 2 à 13, **caractérisé en ce qu'**au moins le tronçon cunéiforme (116, 216, 316, 416, 516, 616, 716, 816, 916, 1016) est fabriqué d'un seul tenant avec le reste du dispositif de fixation et/ou d'éjection (114, 214, 314, 414, 514, 614, 714, 814, 914, 1014), en particulier avec l'élément en forme de tige (125, 225, 325, 425, 525, 625, 725, 825, 925, 1025).

15. Dispositif de lancette de sang selon l'une quelconque des revendications précédentes, **caractérisé en ce que** le corps de base (2, 102, 202, 302, 402, 502, 602, 702, 802) présente au moins une paroi intérieure (240, 440, 640, 740, 840) plane pour le déplacement défini du dispositif de fixation et/ou d'éjection (114, 214, 314, 414, 514, 614, 714, 814, 914, 1014).
